# EUROPEAN PATENT APPLICATION

(11) **EP 3 162 286 A1**
(43) Date of publication of application: **03.05.2017**
(21) Application number: 15812071.7
(22) Date of filing: 22.06.2015
(51) Int. Cl.: A61B 5/026, A61B 5/00

(54) **MEASUREMENT DEVICE AND MEASUREMENT METHOD**

(30) Priority: 24.06.2014 JP 2014129206; 24.10.2014 JP 2014217402
(71) Applicant: Kyocera Corporation, Kyoto-shi, Kyoto 612-8501 (JP)
(72) Inventor: FUJISHIRO, Masato, Kyoto-shi Kyoto 612-8501 (JP)
(74) Representative: Schwabe - Sandmair - Marx
(86) International application number: PCT/JP2015/003116
(87) International publication number: WO 2015/198583

(57) **Abstract**

A measurement apparatus for measuring biological information by contacting a test site to a contact interface (14) includes a contact state detector (11) that detects a contact state of the test site on the contact interface (14), a biological sensor (13) that acquires biological information from the test site, a notification interface (12), and a controller (15) that measures the biological information based on output from the biological sensor (13). The controller (15) judges the contact state of the test site on the contact interface (14) based on output from the contact state detector (11) and provides notification, via a notification interface (12), of information related to the contact state.

## Description

### CROSS-REFERENCE TO RELATED APPLICATION

This application claims priority to and the benefit of Japanese Patent Application No. 2014-129206 filed June 24, 2014, and Japanese Patent Application No. 2014-217402 filed October 24, 2014, the entire contents of which are incorporated herein by reference.

### TECHNICAL FIELD

This disclosure relates to a measurement apparatus and a measurement method.

### BACKGROUND

An example of an existing measurement apparatus measures biological information by acquiring biological output information from a test site, such as a fingertip of a subject (user). For example, a blood flow measurement apparatus that measures blood flow as the biological information irradiates a fingertip with laser light and measures the blood flow based on scattered light from the blood flow in a capillary at the fingertip (for example, see JP H03-21208 Y2 (PTL 1)).

### CITATION LIST

### Patent Literature

PTL 1: JP H03-21208 Y2

### SUMMARY

### (Technical Problem)

The measurement accuracy of the biological information changes depending on the contact state of the test site with respect to the measurement apparatus. It is difficult, however, for a subject that does not have expertise regarding measurement of biological information to place the test site in contact with the measurement apparatus in a suitable state. In order for the measurement apparatus to measure biological information with high accuracy, the subject preferably places the test site in contact in a suitable contact state.

It would therefore be helpful to provide a measurement apparatus and measurement method that can improve the measurement accuracy of biological information.

### (Solution to Problem)

To this end, a measurement apparatus according to this disclosure is for measuring biological information by contacting a test site to a contact interface, the measurement apparatus including:
a contact state detector configured to detect a contact state of the test site on the contact interface;
a biological sensor configured to acquire biological information from the test site;
a notification interface; and
a controller configured to measure the biological information based on output from the biological sensor;
such that the controller judges the contact state of the test site on the contact interface based on output from the contact state detector and provides notification, via the notification interface, of information related to the contact state.

The controller may be further configured to activate the biological sensor upon judging that the contact state is suitable for measuring the biological information.

The information related to the contact state may include information related to pressure with which the test site contacts the contact interface and/or information related to a positional relationship between the test site and the contact interface.

The contact state detector may include an illuminance measurement unit; and
the controller may judge the contact state based on output of information related to an illuminance value measured by the illuminance measurement unit.

A plurality of the illuminance measurement units may be disposed around the biological sensor in the measurement apparatus; and
the controller may judge the contact state based on output of information related to the illuminance value measured by each of the plurality of illuminance measurement units.

The plurality of illuminance measurement units may be disposed to lie on a common circle, with the biological sensor at the center of the circle.

When the illuminance value measured by each of the plurality of illuminance measurement units is equal to or greater than a predetermined illuminance threshold, the controller may provide notification, via the notification interface, indicating to increase pressure with which the test site contacts the contact interface.

When the illuminance value measured by any of the plurality of illuminance measurement units is less than a predetermined illuminance threshold, the controller may identify an illuminance measurement unit, among the plurality of illuminance measurement units, measuring a highest illuminance value and provide notification, via the notification interface, indicating to move the test site towards the identified illuminance measurement unit.

The contact state detector may include a plurality of strain sensors around the biological sensor; and
the controller may judge the contact state based on output of information related to a strain amount of the contact interface detected by each of the plurality of strain sensors.

When the strain amount detected by each of the plurality of strain sensors is less than a predetermined strain amount threshold, the controller may provide notification, via the notification interface, indicating to increase pressure with which the test site contacts the contact interface.

When the strain amount detected by any of the plurality of strain sensors is equal to or greater than a predetermined strain amount threshold, the controller may identify a strain sensor, among the plurality of strain sensors, detecting a smallest strain amount and provide notification, via the notification interface, indicating to move the test site towards the identified strain sensor.

The contact state detector may include a touch panel; and
the controller may judge the contact state based on output of information related to a contact position of the test site, the contact position being detected by the touch panel.

The biological information may include information related to blood flow.

This disclosure may also be implemented as methods substantially corresponding to the above-described measurement apparatuses, and such methods are to be understood as included in the scope of this disclosure.

For example, a measurement method according to this disclosure is for measuring biological information by contacting a test site to a contact interface, the method including:
detecting, with a contact state detector, a contact state of the test site on the contact interface;
judging, with a controller, the contact state of the test site on the contact interface based on output from the contact state detector and providing notification, with the controller via a notification interface, of information related to the contact state;
acquiring, with a biological sensor, biological information from the test site; and
measuring, with the controller, the biological information based on output from the biological sensor.

### (Advantageous Effect)

According to the measurement apparatus and measurement method of this disclosure, the measurement accuracy of biological information can be improved.

### BRIEF DESCRIPTION OF THE DRAWINGS

In the accompanying drawings:
FIG. 1 is a functional block diagram schematically illustrating the structure of a measurement apparatus according to Embodiment 1;
FIGS. 2A and 2B illustrate an example of a usage state of the measurement apparatus according to Embodiment 1;
FIG. 3 is an enlarged perspective view schematically illustrating the positional relationships between an illuminance measurement unit, a biological sensor, and a contact interface in the measurement apparatus according to Embodiment 1;
FIG. 4 is a flowchart illustrating an example of processing, executed by the controller according to Embodiment 1, to adjust the contact position;
FIG. 5 is a flowchart illustrating an example of processing, executed by the controller, to measure biological information;
FIG. 6 is an enlarged perspective view schematically illustrating the positional relationships between an illuminance measurement unit, a biological sensor, and a contact interface in the measurement apparatus according to Embodiment 2;
FIGS. 7A and 7B schematically illustrate examples of contact states of the test site on the contact interface;
FIG. 8 is a flowchart illustrating an example of processing, executed by the controller according to Embodiment 2, to adjust the contact position of the test site;
FIG. 9 is an enlarged perspective view schematically illustrating the positional relationships between a strain sensor, a biological sensor, and a contact interface in the measurement apparatus according to Embodiment 3;
FIGS. 10A and 10B schematically illustrate examples of contact states of the test site on the contact interface;
FIG. 11 is a flowchart illustrating an example of processing, executed by the controller according to Embodiment 3, to adjust the contact position of the test site;
FIG. 12 is an enlarged perspective view schematically illustrating the positional relationships between a touch panel and a biological sensor in the measurement apparatus according to Embodiment 4; and
FIG. 13 is a flowchart illustrating an example of processing, executed by the controller according to Embodiment 4, to adjust the contact position of the test site.

### DETAILED DESCRIPTION

The following describes disclosed embodiments in detail with reference to the drawings.

### Embodiment 1

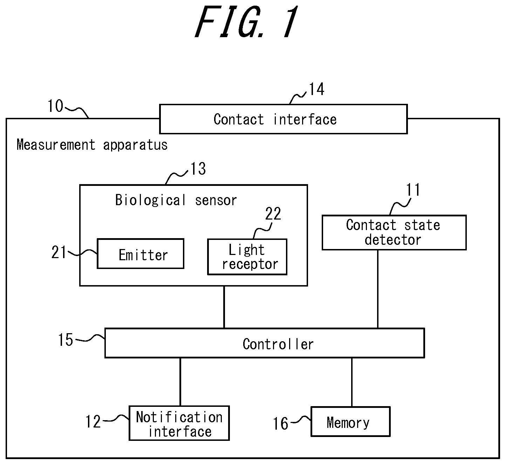

FIG. 1 is a functional block diagram schematically illustrating the structure of a measurement apparatus according to Embodiment 1. This measurement apparatus 10 includes a contact state detector 11, a notification interface 12, a biological sensor 13, a contact interface 14, a controller 15, and a memory 16. In Embodiment 1, the contact state detector 11 is configured by one illuminance measurement unit.

The measurement apparatus 10 may, for example, be an electronic device such as a mobile phone or may be an apparatus exclusively for measuring biological information. In addition to being a mobile phone, the electronic device may be any of a variety of other devices, such as a portable music player, a laptop computer, a wristwatch, a tablet, a game device, or the like. In this disclosure, the measurement apparatus 10 is described as being a mobile phone.

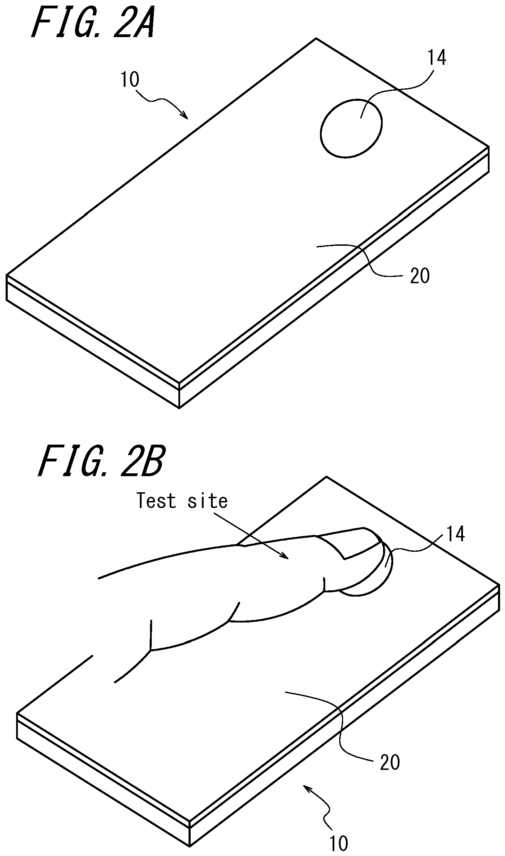

The measurement apparatus 10 measures biological information at a test site in contact with the contact interface 14. FIGS. 2A and 2B illustrate an example of a usage state of the measurement apparatus 10. As illustrated in FIG. 2A, the measurement apparatus 10 includes a contact interface 14 on the back side of a housing 20 of the mobile phone. As illustrated in FIG. 2B, the subject measures biological information with the measurement apparatus 10 while pressing a finger, which is the test site, against the contact interface 14.

The biological information measured by the measurement apparatus 10 may be any biological information that can be measured using the biological sensor 13. In this embodiment, as one example, the measurement apparatus 10 is described as measuring the subject's amount of blood flow, which is information related to blood flow.

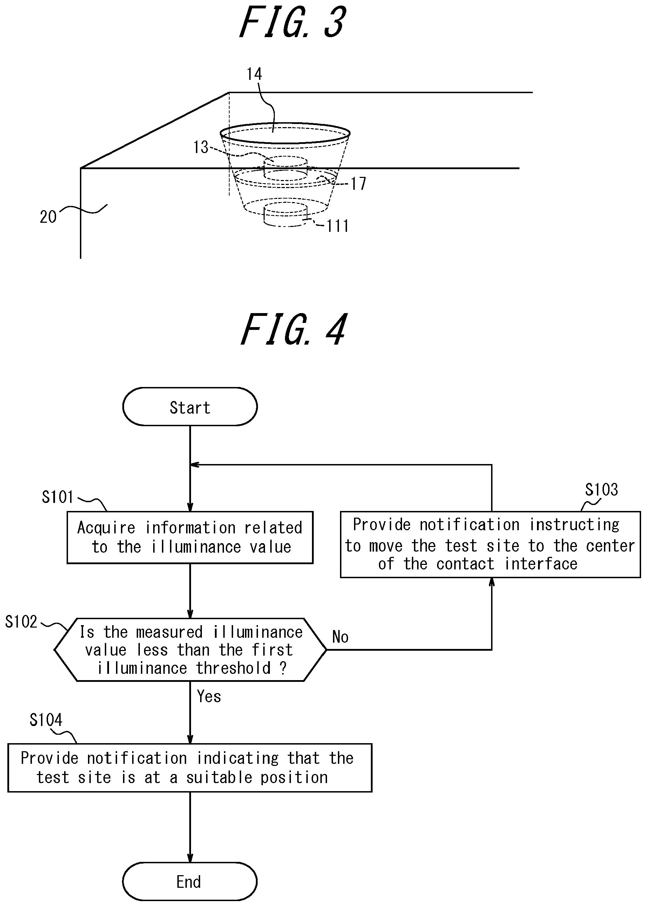

FIG. 3 is an enlarged perspective view schematically illustrating the positional relationships between an illuminance measurement unit 111, the biological sensor 13, and the contact interface 14 in the measurement apparatus 10. In this embodiment, the contact interface 14 is disposed on the back surface of the housing 20 and is transparent and disk-shaped. From the contact interface 14 toward the inside of the housing 20, the biological sensor 13 and the illuminance measurement unit 111 are disposed at a distance in the vertical direction from the back surface. The biological sensor 13 is, for example, disposed on a transparent, disk-shaped base 17 provided between the contact interface 14 and the illuminance measurement unit 111. The illuminance measurement unit 111 measures the illuminance value of external light incident from the contact interface 14. The biological sensor 13 emits measurement light onto the test site in contact with the contact interface 14 and receives scattered light from the test site. The measurement apparatus 10 measures biological information based on the output acquired from the biological sensor 13 (biological measurement output).

In FIG. 1, the illuminance measurement unit 111 that constitutes the contact state detector 11 measures the illuminance value of external light incident from the contact interface 14. The illuminance measurement unit 111 may, for example, be configured by a digital video camera. The illuminance measurement unit 111 may, for example, be configured by an illuminance sensor that uses a phototransistor, a photodiode, or the like. In Embodiment 1, the illuminance measurement unit 111 is described as being a digital video camera. Information related to the illuminance value measured by the illuminance measurement unit 111 is transmitted to the controller 15 and is used in the controller 15 to judge the contact state of the test site on the contact interface 14.

Based on control by the controller 15, the notification interface 12 provides notification of information related to the contact state of the test site on the contact interface 14. The information related to the contact state for example includes information related to the positional relationship between the test site and the contact interface 14. The measurement accuracy of the amount of blood flow may change depending on the positional relationship between the biological sensor 13 and the test site. Accordingly, the subject preferably places the test site at a suitable position relative to the biological sensor 13 so that the error in the measurement result of the amount of blood flow falls within a predetermined error range. Based on the positional relationship between the biological sensor 13 and the contact interface 14, the notification interface 12 provides notification so that the subject can place the test site on the contact interface 14 at a suitable position relative to the biological sensor 13.

The notification interface 12 can provide notification for example by a visual method using an image, characters, light emission, or the like; an auditory method using audio or the like; or a combination of these methods. In the case of providing notification with a visual method, the notification interface 12 for example is a display device that provides notification by displaying images or characters. The notification interface 12 may, for example, provide notification by causing an LED or other such light emitting device to emit light. In the case of providing notification with an auditory method, the notification interface 12 for example is a sound generating device, such as a speaker, that provides notification by outputting an alarm sound, audio guidance, or the like. Provision of notification by the notification interface 12 is not limited to a visual or auditory method. Any method recognizable by the subject may be adopted. Specific control of the notification interface 12 by the controller 15 is described below.

The biological sensor 13 acquires biological information from the test site. When the measurement apparatus 10 measures the amount of blood flow, as in this embodiment, then the biological sensor 13 includes an emitter 21 and a light receiver 22.

The emitter 21 emits laser light based on control by the controller 15. The emitter 21 emits laser light onto the test site as measurement light, the laser light having a wavelength capable of detecting a predetermined component included in blood. The emitter 21 may, for example, be configured by a Laser Diode (LD).

The light receiver 22 receives scattered measurement light from the test site as biological information. The light receiver 22 may, for example, be configured by a photodiode (PD). The biological sensor 13 transmits a photoelectric conversion signal (biological measurement output) of the scattered light received by the light receiver 22 to the controller 15.

The contact interface 14 is a portion of the measurement apparatus 10 that contacts the test site, such as a finger, in order for the subject to measure biological information. The contact interface 14 is, for example, configured by a plate-shaped member. The contact interface 14 is configured by a member that is transparent at least with respect to the measurement light from the emitter 21 and the scattered light from the test site that is in contact.

The controller 15 is a processor that, starting with the functional blocks of the measurement apparatus 10, controls and manages the measurement apparatus 10 overall. The controller 15 is configured using a processor such as a Central Processing Unit (CPU) that executes a program prescribing control procedures. Such a program may, for example, be stored in the memory 16, in an external storage medium, or the like.

Based on the output from the contact state detector 11, the controller 15 judges the contact state of the test site on the contact interface 14. In this embodiment, the controller 15 judges the contact state of the test site on the contact interface 14 based on the information related to the illuminance value measured by the illuminance measurement unit 111. If the illuminance measurement unit 111, biological sensor 13, and contact interface 14 are in the positional relationships illustrated in FIG. 3, then when the test site is in contact with the contact interface 14 at the center of the contact interface 14, the test site is positioned in front of the biological sensor 13, and the measurement accuracy of the amount of blood flow is thought to be high. As the location where the test site contacts the contact interface 14 moves farther away from the center, the measurement accuracy of the amount of blood flow is thought to be reduced. Also, as the location where the test site contacts the contact interface 14 moves farther away from the center, the test site approaches the periphery of the contact interface 14, and the area in which the test site covers the contact interface 14 reduces. Therefore, the illuminance value of external light incident on the illuminance measurement unit 111 from the contact interface 14 increases. Based on this fact, the controller 15 judges that the test site is at a suitable position for measuring the amount of blood flow when the illuminance value measured by the illuminance measurement unit 111 is less than a first illuminance threshold, i.e. when the test site is at a position closer to the center than a predetermined range in the contact interface 14. Conversely, the controller 15 judges that the test site is not at a suitable position when the illuminance value measured by the illuminance measurement unit 111 is equal to or greater than the first illuminance threshold.

The controller 15 provides notification via the notification interface 12 of information related to the judged contact state. For example, upon judging that the test site is at a suitable position, the controller 15 may provide notification, via the notification interface 12, indicating that the test site is at a suitable position. In this case, the controller 15 may provide notification, via the notification interface 12, indicating the start of measurement of the amount of blood flow.

On the other hand, upon judging that the test site is not at a suitable position, the controller 15 may provide notification, via the notification interface 12, indicating that the test site is not at a suitable position. The controller 15 may provide notification, via the notification interface 12, instructing the subject to move the test site to the center of the contact interface 14. After recognizing the notification, the subject can change the position of the test site relative to the contact interface 14, thereby adjusting the test site to be at a suitable position.

The controller 15 activates the biological sensor 13 upon judging that the contact state is suitable for measurement of biological information. In the above-described example, the controller 15 activates the biological sensor 13 upon judging that the test site is at a suitable position. The activated biological sensor 13 acquires biological information.

The controller 15 then measures the biological information based on the biological measurement output from the biological sensor 13. In greater detail, the controller 15 generates biological information based on the output from the light receiver 22.

A technique for the controller 15 to measure the amount of blood flow using the Doppler shift is now described. When measuring the amount of blood flow, the controller 15 emits laser light from the emitter 21 onto body tissue (the test site) and receives scattered light that is scattered from the body tissue with the light receiver 22. The controller 15 then calculates the amount of blood flow based on output related to the scattered light that was received.

In the body tissue, scattered light that is scattered from moving blood cells undergoes a frequency shift (Doppler shift), due to the Doppler effect, relative to the speed of travel of the blood cells within the blood. The controller 15 detects the beat signal due to interference between scattered light from still tissue and the scattered light from moving blood cells. This beat signal represents strength as a function of time. The controller 15 then turns the beat signal into a power spectrum that represents power as a function of frequency. In this power spectrum of the beat signal, the Doppler shift frequency is proportional to the speed of blood cells, and the power corresponds to the amount of blood cells. The controller 15 calculates the amount of blood flow by multiplying the power spectrum of the beat signal by the frequency and integrating.

The controller 15 determines whether the acquisition of the biological information by the biological sensor 13 is complete. The controller 15 may, for example, judge that acquisition of the biological information is complete once a predetermined length of time elapses after the biological sensor 13 starts to acquire the biological information. The controller 15 may also, for example, judge that acquisition of the biological information is complete once the biological sensor 13 has acquired sufficient biological information to measure the biological information.

Upon judging that acquisition of biological information is complete, the controller 15 suspends emission of laser light from the emitter 21.

The controller 15 may display the measured biological information on a display device provided in the measurement apparatus 10, such a display device being configured by a known display such as a liquid crystal display, an organic EL display, an inorganic EL display, or the like.

The memory 16 may be configured with a semiconductor memory, a magnetic memory, or the like. The memory 16 stores a variety of information, programs for causing the measurement apparatus 10 to operate, and the like and also functions as a working memory. The memory 16 for example stores the first illuminance threshold that serves as a reference for the controller 15 to judge the position of the test site.

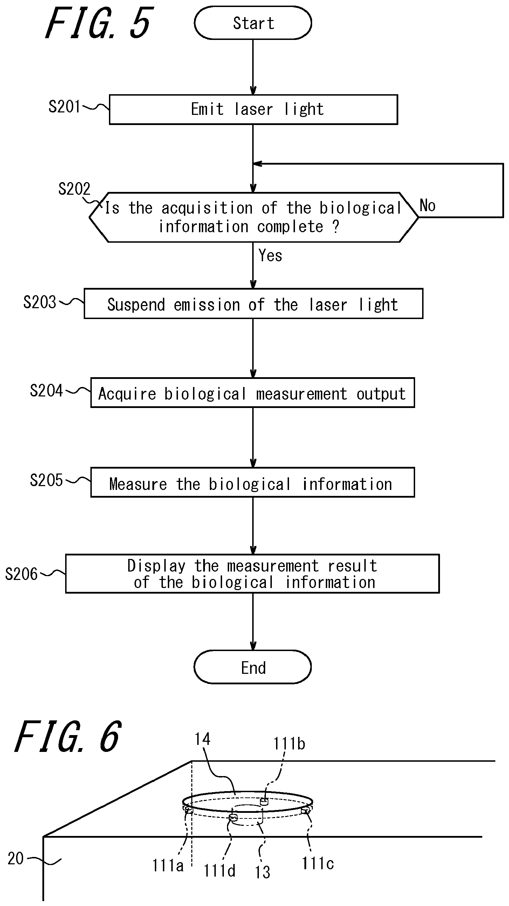

Next, with reference to the flowcharts in FIGS. 4 and 5, an example of processing executed by the controller 15 in Embodiment 1 is described. FIG. 4 is a flowchart illustrating an example of processing (contact position adjustment processing), executed by the controller 15, to adjust the contact position of the test site. FIG. 5 is a flowchart illustrating an example of processing, executed by the controller 15, to measure biological information. The controller 15 begins the processing flow in FIG. 4 for example when the measurement apparatus 10 is placed in a state capable of measuring the biological information by the subject operating the measurement apparatus 10.

The controller 15 acquires information related to the illuminance value measured by the illuminance measurement unit 111 (step S101).

The controller 15 judges whether the measured illuminance value is less than the first illuminance threshold by referring to the memory 16 (step S102).

When judging that the measured illuminance value is equal to or greater than the first illuminance threshold (step S102: No), the controller 15 judges that the test site is not at a suitable position on the contact interface 14 and provides notification, via the notification interface 12, instructing the subject to move the test site to the center of the contact interface 14 (step S103). After recognizing the notification, the subject changes the position of the test site relative to the contact interface 14, thereby adjusting the test site to be at a suitable position. Processing then transitions to step S101.

When judging that the measured illuminance value is less than the first illuminance threshold (step S102: Yes), the controller 15 judges that the test site is at a suitable position on the contact interface 14.

The controller 15 provides notification, via the notification interface 12, indicating that the test site is at a suitable position on the contact interface 14 (step S104) and terminates the processing.

Once the contact position of the test site is adjusted by the processing in FIG. 4, the controller 15 starts the processing for measurement illustrated in FIG. 5 and measures the biological information.

The controller 15 emits laser light from the emitter 21 (step S201). By emission of laser light, acquisition of biological information by the biological sensor 13 begins.

The controller 15 judges whether the acquisition of the biological information by the biological sensor 13 is complete (step S202).

When judging that acquisition of the biological information is not complete (step S202: No), the controller 15 repeats step S202 until judging that acquisition of the biological information is complete.

When judging that acquisition of biological information is complete (step S202: Yes), the controller 15 suspends emission of laser light from the emitter 21 (step S203).

Next, the controller 15 acquires output related to the biological information acquired by the biological sensor 13, i.e. acquires the biological measurement output from the biological sensor 13 (step S204).

The controller 15 measures the biological information based on the biological measurement output acquired from the biological sensor 13 (step S205).

The controller 15 displays the measurement result of the measured biological information using the display device (step S206). The subject can learn the amount of blood flow by confirming the displayed measurement result.

In this way, in the measurement apparatus 10 according to Embodiment 1, the controller 15 judges the contact state of the test site on the contact interface 14 based on the illuminance value measured by the illuminance measurement unit 111. Upon judging that the test site is not at a suitable position on the contact interface 14 for measuring biological information, the controller 15 notifies the subject of information related to the contact state. Therefore, the subject can adjust the position of the test site in accordance with the notification, making it easier to adjust the contact state of the test site to a position suitable for measuring the biological information. In this way, the measurement apparatus 10 can improve the measurement accuracy of the biological information.

Furthermore, the controller 15 causes laser light to be emitted from the emitter 21 when the test site is at a suitable position and suspends emission of laser light when acquisition of biological information is complete. As a result, the controller 15 can suppress unnecessary power consumption by the measurement apparatus 10.

### Embodiment 2

FIG. 6 is an enlarged perspective view schematically illustrating the positional relationships between the contact state detector 11, the biological sensor 13, and the contact interface 14 in the measurement apparatus 10 according to Embodiment 2. In Embodiment 2, the contact state detector 11 is provided with a plurality of illuminance measurement units 111 that are, for example, configured by illuminance sensors. The illuminance measurement units 111 are disposed around the biological sensor 13. The following describes the differences from Embodiment 1, omitting a description of common features.

In Embodiment 2, as in Embodiment 1, the contact interface 14 is disposed on the back surface of the housing 20 of a mobile phone and is transparent and disk-shaped. In Embodiment 2, the biological sensor 13 is disposed at a position opposite the center of the contact interface 14. Around the periphery of the contact interface 14, four illuminance measurement units 111a, 111b, 111c, and 111d are disposed at equal intervals. When not distinguishing between the four illuminance measurement units 111a, 111b, 111c, and 111d, these units are referred to below as illuminance measurement units 111. The quantity and arrangement of the illuminance measurement units 111 illustrated in FIG. 6 are only examples. Any number of illuminance measurement units 111 may be provided in the measurement apparatus 10. Furthermore, the illuminance measurement units 111 may be disposed at any positions that allow judgment of whether the position on the contact interface 14 contacted by the test site is suitable for measurement of the biological information in terms of the positional relationship with the biological sensor 13. Positions that allow judgment of suitability for measurement of the biological information are, for example, in the vicinity of the biological sensor 13. For example, the number and arrangement of the illuminance measurement units 111 is not limited to four illuminance measurement units 111 disposed at equal intervals, as described above, and may be changed as necessary. Numerous illuminance measurement units 111 may, for example, be disposed on the side where the base of the finger is positioned when the subject contacts the finger, which is the test site, to the contact interface 14, and the controller 15 may control the various functional units to cause the subject to place the test site in contact suitably with the contact interface 14.

In addition to the information related to the positional relationship between the test site and the contact interface 14, in Embodiment 2, the notification interface 12 for example also provides notification of information related to the pressure with which the test site contacts the contact interface 14 as the information related to the contact state. The pressure with which the test site contacts the contact interface 14 is, for example, preferably included in a suitable pressure range such that, based on the statistical relationship between pressure and error in measurement of the amount of blood flow, the error in the measurement result of the amount of blood flow falls within a predetermined error range. The notification interface 12 provides notification so that the subject can adjust the pressure of the test site on the contact interface 14 to be within a suitable pressure range.

In Embodiment 2, the controller 15 judges the contact state of the test site on the contact interface 14 based on the information related to illuminance values measured by the various illuminance measurement units 111. For example, when the illuminance values measured by the various illuminance measurement units 111 are all less than a second illuminance threshold, the controller 15 judges that the test site is in a state suitable for measurement of the amount of blood flow. The reason is that when the illuminance values measured by the four illuminance measurement units 111 are all less than the second illuminance threshold, the test site is covering the biological sensor 13 positioned at the center of the four illuminance measurement units 111, and the positional relationship between the biological sensor 13 and the test site can be judged to be suitable for measurement of the amount of blood flow. When judging that the test site is in a suitable state for measurement of the amount of blood flow, the controller 15 may provide notification, via the notification interface 12, indicating that the test site is at a suitable position. In this case, the controller 15 may provide notification, via the notification interface 12, indicating the start of measurement of the amount of blood flow.

When at least one illuminance value measured by the illuminance measurement units 111 is equal to or greater than the second illuminance threshold, the controller 15 judges that the test site is not in a state suitable for measurement of the amount of blood flow. In this case, the controller 15 can make a more detailed judgment of the contact state of the test site by judging whether the illuminance values measured by the illuminance measurement units 111 are equal to or greater than a third illuminance threshold. For example, for the contact pressure with which the test site contacts the contact interface 14, there may be an appropriate pressure range suitable for measurement of the amount of blood flow. When the contact pressure on the contact interface 14 is too weak, for example, then the measurement apparatus 10 might not be able to measure the biological information accurately because of the effect of noise. Based on the output of the illuminance measurement units 111, the controller 15 can judge the state of contact pressure. Furthermore, based on the output of the illuminance measurement units 111, the controller 15 for example can judge the direction in which the test site is preferably moved. The following describes the detailed judgment made by the controller 15. The third illuminance threshold may be set to an appropriate value equal to or greater than the second illuminance threshold.

For example when the illuminance values measured by the various illuminance measurement units 111 are all equal to or greater than the third illuminance threshold, the controller 15 can judge that the test site is at the center of the four illuminance measurement units 111, but that the contact pressure with which the test site contacts the contact interface 14 is weak. The reason is that when the illuminance values measured by the various illuminance measurement units 111 are all equal to or greater than the third illuminance threshold, all of the illuminance measurement units 111 are insufficiently covered by the test site. In this case, the controller 15 provides notification, via the notification interface 12, indicating to contact the test site more firmly to the contact interface 14. When the subject increases the contact pressure after confirming the notification, and the illuminance values measured by the illuminance measurement units 111 fall below the third illuminance threshold, the controller 15 judges that the contact pressure is sufficiently strong for measuring the biological information.

For example, when at least one of the measured illuminance values of the illuminance measurement units 111 is less than the third illuminance threshold, each of the illuminance measurement unit(s) 111 that measured an illuminance value less than the third illuminance threshold is covered by the test site. Therefore, the controller 15 judges that an illuminance value less than the third illuminance threshold was measured. In this case, among the four illuminance measurement units 111, the controller 15 identifies the illuminance measurement unit 111 measuring the highest illuminance value.

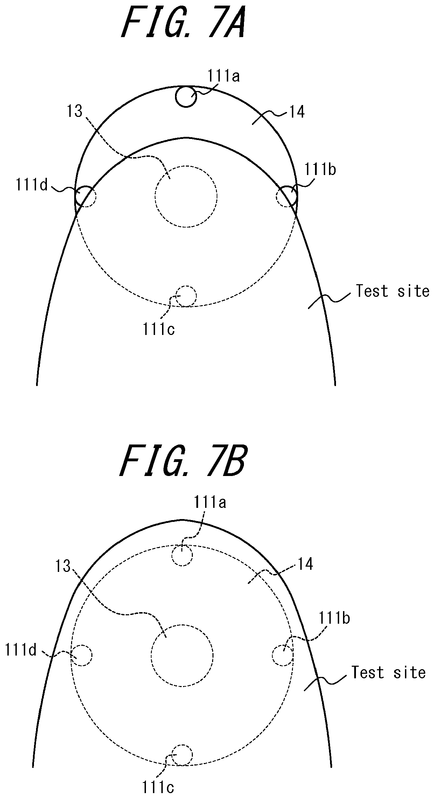

FIGS. 7A and 7B schematically illustrate examples of contact states of the test site on the contact interface 14. FIGS. 7A and 7B show the contact state of the test site as seen from the back side of the housing 20. Only the illuminance measurement units 111, the biological sensor 13, the contact interface 14, and the test site are illustrated. For example, in the state illustrated in FIG. 7A, among the four illuminance measurement units 111a, 111b, 111c, and 111d, the illuminance measurement unit 111c is covered by the test site and therefore measures the lowest illuminance value. The illuminance measurement units 111b and 111d are partially covered by the test site and therefore measure illuminance values corresponding to external light that is incident from the portion of the contact interface 14 not covered by the test site. The illuminance measurement unit 111a is not covered by the test site and measures the highest illuminance value among the four illuminance measurement units 111a, 111b, 111c, and 111d. In this way, the illuminance measurement unit 111a that measures the highest illuminance value is thought not to be covered by the test site, or depending on the position of the test site, is thought to have the smallest area covered by the test site among the four illuminance measurement units 111. Therefore, the controller 15 judges that the test site contacts the contact interface 14 off-center in the direction of the illuminance measurement unit 111c, which is in the opposite direction from the identified illuminance measurement unit 111a across the biological sensor 13. In this case, the controller 15 provides notification, via the notification interface 12, indicating to move the test site in the direction of the identified illuminance measurement unit 111a across the biological sensor 13. When the subject adjusts the position of the test site after confirming the notification, for example so as to arrive at the state illustrated in FIG. 7B, then the difference in the illuminance values measured by the various illuminance measurement units 111 decreases, and the controller 15 can judge that the test site has been adjusted to move to the center of the contact interface 14.

When at least one of the measured illuminance values of the illuminance measurement units 111 is less than the third illuminance threshold, the controller 15 may for example calculate the direction and the distance to move the test site to achieve a suitable contact state by using a predetermined algorithm to perform weighting based on the illuminance values measured by the various illuminance measurement units 111. For example, in the example in FIG. 7A, the controller 15 performs weighting based on the illuminance values measured by the four illuminance measurement units 111a, 111b, 111c, and 111d and calculates the direction and the distance to move the test site to achieve the contact state illustrated in FIG. 7B. The controller 15 provides notification of the calculated result via the notification interface 12. When the subject adjusts the position of the test site after confirming the notification so that the difference in the illuminance values measured by the various illuminance measurement units 111 decreases, then the controller 15 can judge that the test site has been adjusted to move to the center of the contact interface 14.

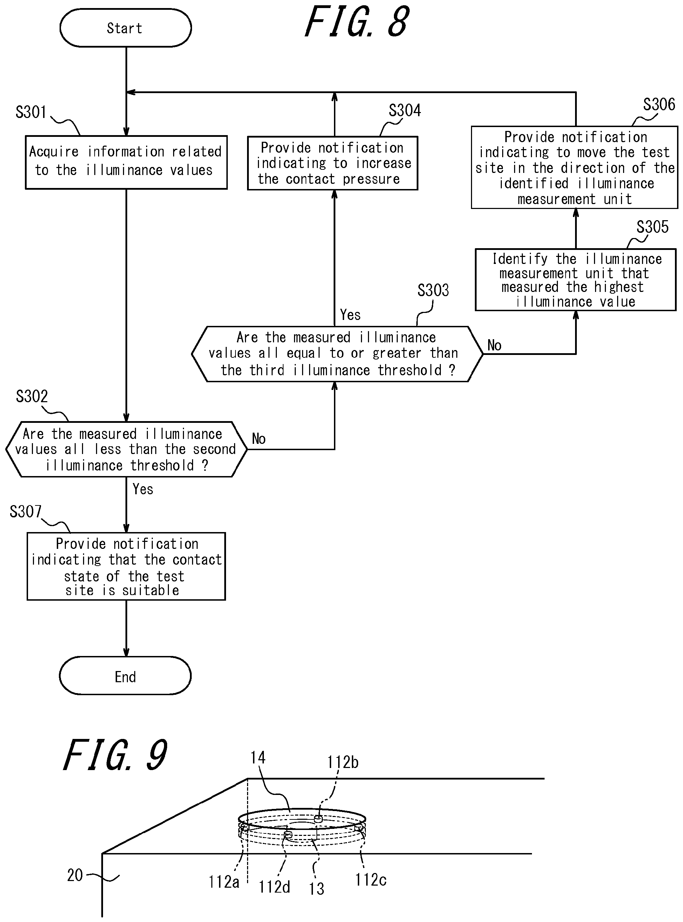

Next, with reference to the flowchart in FIG. 8, an example of the contact position adjustment processing that is executed by the controller 15 in Embodiment 2 is described. The controller 15 begins the processing flow in FIG. 8 for example when the measurement apparatus 10 is placed in a state capable of measuring the biological information by the subject operating the measurement apparatus 10.

The controller 15 acquires information related to the illuminance values measured by the four illuminance measurement units 111 (step S301).

The controller 15 judges whether the illuminance values measured by the four illuminance measurement units 111 are all less than the second illuminance threshold (step S302).

When judging that at least one of the illuminance values is equal to or greater than the second illuminance threshold (step S302: No), the controller 15 then judges whether the illuminance values measured by the four illuminance measurement units 111 are all equal to or greater than the third illuminance threshold (step S303).

When judging that the illuminance values measured by the four illuminance measurement units 111 are all equal to or greater than the third illuminance threshold (step S303: Yes), the controller 15 judges that the contact pressure of the test site on the contact interface 14 is weak and provides notification, via the notification interface 12, indicating to increase the contact pressure (step S304). Processing then transitions to step S301.

When judging that at least one of the illuminance values is less than the third illuminance threshold (step S303: No), the controller 15 judges that the test site is not contacting the center of the contact interface 14 and identifies the illuminance measurement unit 111, among the four illuminance measurement units 111, that measured the highest illuminance value (step S305).

The controller 15 then provides notification, via the notification interface 12, indicating to move the test site in the direction of the illuminance measurement unit 111 identified as measuring the highest illuminance value (step S306). Processing then transitions to step S301.

Upon judging, in step S302, that the illuminance values measured by the four illuminance measurement units 111 are all less than the second illuminance threshold (step S302: Yes), the controller 15 provides notification, via the notification interface 12, indicating that the contact state of the test site is suitable (step S307) and terminates the processing.

Once the contact position of the test site is adjusted by the processing in FIG. 8, the controller 15 starts the processing for measurement illustrated in FIG. 5 and measures the biological information.

In this way, in the measurement apparatus 10 according to Embodiment 2, the controller 15 judges the contact state of the test site on the contact interface 14 based on the illuminance values measured by the plurality of illuminance measurement units 111. When judging that the contact state is not suitable, the controller 15 judges whether the contact pressure is weak, whether the position of the test site is not suitable, and so forth based on the illuminance values measured by the illuminance measurement units 111 and then provides notification of the result of judgment. Furthermore, when judging that the position of the test site is not suitable, the controller 15 can provide notification of the direction in which to move the test site. Therefore, the subject can adjust the position of the test site in accordance with the notification, making it easier to adjust the contact state of the test site to a position suitable for measuring the biological information. In this way, the measurement apparatus 10 can improve the measurement accuracy of the biological information.

### Embodiment 3

FIG. 9 is an enlarged perspective view schematically illustrating the positional relationships between the contact state detector 11, the biological sensor 13, and the contact interface 14 in the measurement apparatus 10 according to Embodiment 3. In Embodiment 3, the contact state detector 11 is, for example, provided with a plurality of strain sensors. The strain sensors are disposed around the biological sensor 13.

In Embodiment 3, the contact interface 14 is disposed on the back surface of the housing 20 of a mobile phone and is transparent and disk-shaped. In this embodiment, the contact interface 14 is configured by a flexible member. Around the periphery of the contact interface 14, four strain sensors 112a, 112b, 112c, and 112d are disposed at equal intervals. When not distinguishing between the four strain sensors 112a, 112b, 112c, and 112d, these sensors are referred to below as strain sensors 112. The measurement apparatus 10 in this embodiment includes a support plate 18 that supports the biological sensor 13 inside the housing 20. The support plate 18 is configured by a member that is transparent at least with respect to the measurement light from the emitter 21 and the scattered light from the test site that is in contact. The biological sensor 13 is disposed at a position opposite the center of the support plate 18.

When the subject contacts the test site to the contact interface 14, the strain sensors 112 detect strain occurring in the contact interface 14 because of pressure on the contact interface 14. Each of the strain sensors 112 may, for example, be configured by a piezoelectric element that detects information, in the form of voltage, related to strain occurring due to pressure. The piezoelectric element may be a unimorph, bimorph, or laminated piezoelectric element. The strain sensors 112 are not limited to being piezoelectric elements. Any strain sensor that can detect strain of the contact interface 14 may be used, such as a semiconductor strain sensor, resistance strain sensor, or the like.

The quantity and arrangement of the strain sensors 112 illustrated in FIG. 9 are only examples. Any number of strain sensors 112 may be provided in the measurement apparatus 10. Furthermore, the strain sensors 112 may be disposed at any positions that allow judgment of whether the position on the contact interface 14 contacted by the test site is suitable for measurement of the biological information in terms of the positional relationship with the biological sensor 13. Positions that allow judgment of suitability for measurement of the biological information are, for example, in the vicinity of the biological sensor 13. For example, the number and arrangement of the strain sensors 112 is not limited to four strain sensors 112 disposed at equal intervals, as described above, and may be changed as necessary. Numerous strain sensors 112 may, for example, be disposed on the side where the base of the finger is positioned when the subject contacts the finger, which is the test site, to the contact interface 14, and the controller 15 may control the various functional units to cause the subject to place the test site, such as a finger, in contact suitably with the contact interface 14.

As the information related to the contact state, in this embodiment, the notification interface 12 provides notification of information related to the positional relationship between the test site and the contact interface 14 and information related to the pressure with which the test site contacts the contact interface 14. The pressure with which the test site contacts the contact interface 14 is, for example, preferably included in a suitable pressure range such that, based on the statistical relationship between pressure and error in measurement of the amount of blood flow, the error in the measurement result of the amount of blood flow falls within a predetermined error range. The notification interface 12 provides notification so that the subject can adjust the pressure of the test site on the contact interface 14 to be within a suitable pressure range.

In this embodiment, the controller 15 judges the contact state of the test site on the contact interface 14 based on the information related to strain detected by the various strain sensors 112. For example, when the strain amounts detected by the various strain sensors 112 are all equal to or greater than a first strain amount threshold, the controller 15 judges that the test site is in a state suitable for measurement of the amount of blood flow. The reason is that when the strain amounts detected by the four strain sensors 112 are all equal to or greater than the first strain amount threshold, the test site is covering the biological sensor 13 positioned at the center of the four strain sensors 112, and the positional relationship between the biological sensor 13 and the test site can be judged to be suitable for measurement of the amount of blood flow. When judging that the test site is in a suitable state for measurement of the amount of blood flow, the controller 15 may provide notification, via the notification interface 12, indicating that the test site is at a suitable position. In this case, the controller 15 may provide notification, via the notification interface 12, indicating the start of measurement of the amount of blood flow.

When at least one of the strain amounts detected by the strain sensors 112 is less than the first strain amount threshold, the controller 15 judges that the test site is not in a state suitable for measurement of the amount of blood flow. In this case, the controller 15 can make a more detailed judgment of the contact state of the test site by judging whether the strain amounts detected by the strain sensors 112 are less than a second strain amount threshold. For example, for the contact pressure with which the test site contacts the contact interface 14, there may be an appropriate pressure range suitable for measurement of the amount of blood flow. When the contact pressure on the contact interface 14 is too weak, for example, then the measurement apparatus 10 might not be able to measure the biological information accurately because of the effect of noise. Based on the output of the strain sensors 112, the controller 15 can judge the state of contact pressure. Furthermore, based on the output of the strain sensors 112, the controller 15 for example can judge the direction in which the test site is preferably moved. The following describes the detailed judgment made by the controller 15. The second strain amount threshold may be set to an appropriate value equal to or less than the first strain amount threshold.

For example, when the strain amounts detected by the strain sensors 112 are all less than the second strain amount threshold, the controller 15 can judge that the test site is at the center of the four strain sensors 112 but that the contact pressure with which the test site contacts the contact interface 14 is weak. The reason is that when the strain amounts detected by the strain sensors 112 are all less than the second strain amount threshold, all of the strain sensors 112 are insufficiently pressed by the test site. In this case, the controller 15 provides notification, via the notification interface 12, indicating to contact the test site more firmly to the contact interface 14. When the subject increases the contact pressure after confirming the notification, and the strain amounts detected by the strain sensors 112 become equal to or greater than the second strain amount threshold, the controller 15 judges that the contact pressure is sufficiently strong for measuring the biological information.

For example, when at least one of the detected strain amounts of the strain sensors 112 is equal to or greater than the second strain amount threshold, each of the strain sensor(s) 112 that detected a strain amount equal to or greater than the second strain amount threshold is pressed by the test site. Therefore, the controller 15 judges that a strain amount equal to or greater than the second strain amount threshold was detected. In this case, among the four strain sensors 112, the controller 15 identifies the strain sensor 112 detecting the lowest strain amount.

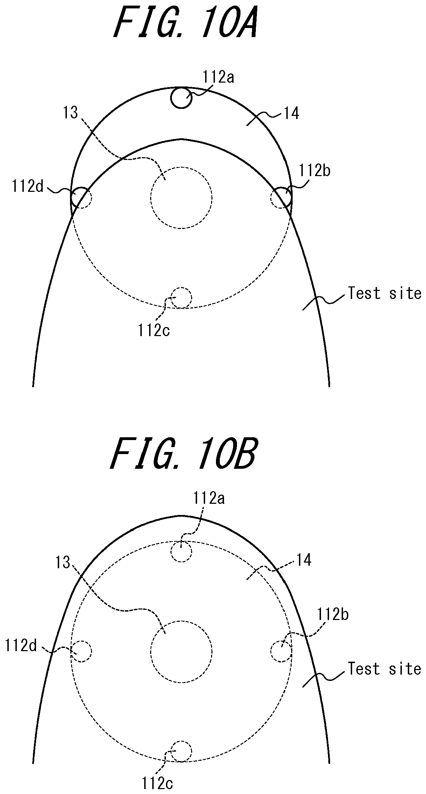

FIGS. 10A and 10B schematically illustrate examples of contact states of the test site on the contact interface 14. FIGS. 10A and 10B show the contact state of the test site as seen from the back side of the housing 20. Only the strain sensors 112, the biological sensor 13, the contact interface 14, and the test site are illustrated. For example, in the state illustrated in FIG. 10A, among the four strain sensors 112a, 112b, 112c, and 112d, the strain sensor 112c is pressed by the test site and therefore detects the largest strain amount. The strain sensors 112b and 112d detect a strain amount due to pressing of the test site, but the pressing force from the test site on the strain sensors 112b and 112d is smaller than the pressing force on the strain sensor 112c. Therefore, the strain amount detected by the strain sensors 112b and 112d becomes smaller than the strain amount detected by the strain sensor 112c. The strain sensor 112a is not pressed by the test site and therefore does not detect strain. In this way, the strain sensor 112a detecting the smallest strain amount is either not being pressed by the test site or is subjected to the smallest pressing force among the four strain sensors 112. Therefore, the controller 15 judges that the test site contacts the contact interface 14 off-center in the direction of the strain sensor 112c, which is in the opposite direction from the identified strain sensor 112a across the biological sensor 13. In this case, the controller 15 provides notification, via the notification interface 12, indicating to move the test site in the direction of the identified strain sensor 112a across the biological sensor 13. When the subject adjusts the position of the test site after confirming the notification, for example so as to arrive at the state illustrated in FIG. 10B, then the difference in the strain amounts detected by the various strain sensors 112 decreases, and the controller 15 can judge that the test site has been adjusted to move to the center of the contact interface 14.

When at least one of the detected strain amounts of the strain sensors 112 is equal to or greater than the second strain amount threshold, the controller 15 may for example calculate the direction and the distance to move the test site to achieve a suitable contact state by using a predetermined algorithm to perform weighting based on the strain amounts detected by the various strain sensors 112. For example, in the example in FIG. 10A, the controller 15 performs weighting based on the strain amounts detected by the four strain sensors 112a, 112b, 112c, and 112d and calculates the direction and the distance to move the test site to achieve the contact state illustrated in FIG. 10B. The controller 15 provides notification of the calculated result via the notification interface 12. When the subject adjusts the position of the test site after confirming the notification so that the difference in the strain amounts detected by the various strain sensors 112 decreases, then the controller 15 can judge that the test site has been adjusted to move to the center of the contact interface 14.

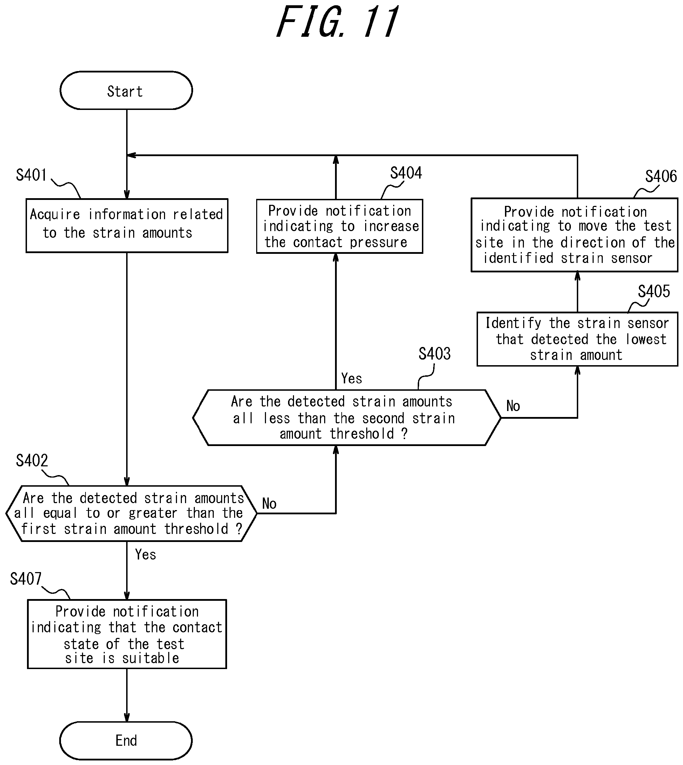

Next, with reference to the flowchart in FIG. 11, an example of the contact position adjustment processing that is executed by the controller 15 in Embodiment 3 is described. The controller 15 begins the processing flow in FIG. 11 for example when the measurement apparatus 10 is placed in a state capable of measuring the biological information by the subject operating the measurement apparatus 10.

The controller 15 acquires information related to the strain amounts detected by the four strain sensors 112 (step S401).

The controller 15 judges whether the strain amounts detected by the four strain sensors 112 are all equal to or greater than the first strain amount threshold (step S402).

When judging that at least one of the strain amounts is less than the first strain amount threshold (step S402: No), the controller 15 then judges whether the strain amounts detected by the four strain sensors 112 are all less than the second strain amount threshold (step S403).

When judging that the strain amounts detected by the four strain sensors 112 are all less than the second strain amount threshold (step S403: Yes), the controller 15 judges that the contact pressure of the test site on the contact interface 14 is weak and provides notification, via the notification interface 12, indicating to increase the contact pressure (step S404). Processing then transitions to step S301.

When judging that at least one of the strain amounts is equal to or greater than the second strain amount threshold (step S403: No), the controller 15 judges that the test site is not contacting the center of the contact interface 14 and identifies the strain sensor 112, among the four strain sensors 112, that detected the lowest strain amount (step S405).

The controller 15 then provides notification, via the notification interface 12, indicating to move the test site in the direction of the strain sensor 112 identified as detecting the smallest strain amount (step S406). Processing then transitions to step S401.

Upon judging, in step S402, that the strain amounts detected by the four strain sensors 112 are all equal to or greater than the first strain amount threshold (step S402: Yes), the controller 15 provides notification, via the notification interface 12, indicating that the contact state of the test site is suitable (step S407) and terminates the processing.

Once the contact position of the test site is adjusted by the processing in FIG. 11, the controller 15 starts the processing for measurement illustrated in FIG. 5 and measures the biological information.

In this way, in the measurement apparatus 10 according to Embodiment 3, the controller 15 judges the contact state of the test site on the contact interface 14 based on the strain amounts detected by the strain sensors 112. When judging that the contact state is not suitable, the controller 15 judges whether the contact pressure is weak, whether the position of the test site is not suitable, and so forth based on the strain amounts detected by the strain sensors 112 and then provides notification of the result of judgment. Furthermore, when judging that the position of the test site is not suitable, the controller 15 can provide notification of the direction in which to move the test site. Therefore, the subject can adjust the position of the test site in accordance with the notification, making it easier to adjust the contact state of the test site to a position suitable for measuring the biological information. In this way, the measurement apparatus 10 can improve the measurement accuracy of the biological information.

### Embodiment 4

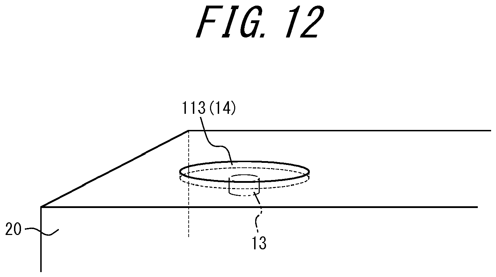

FIG. 12 is an enlarged perspective view schematically illustrating the positional relationships between the contact state detector 11 and the biological sensor 13 in the measurement apparatus 10 according to Embodiment 4. In Embodiment 4, the contact state detector 11 is configured by a touch panel. In this embodiment, the touch panel 113 also functions as the contact interface 14 to which the subject contacts the test site when using the measurement apparatus 10 to measure biological information.

The touch panel 113 detects contact by the subject's test site on the touch surface. As the information related to the contact state, the touch panel 113 detects the region contacted by the test site on the touch surface as the contact position. The touch panel 113 notifies the controller 15 of information related to the detected contact position of the test site.

In this embodiment, as illustrated in FIG. 12, the disk-shaped touch panel 113 is disposed on the back surface of the housing 20 of a mobile phone. The touch panel 113 may be of a well-known type, such as a resistive film type, capacitive type, optical type, or the like. The touch panel 113 is configured by a member that is transparent at least with respect to the measurement light from the emitter 21 and the scattered light from the test site that is in contact.

In this embodiment, based on the information related to the detected contact position of the test site output by the touch panel 113, the controller 15 judges whether the contact position of the test site is at a suitable position for measuring the amount of blood flow. The controller 15 judges that the test site is at a suitable position for measuring the amount of blood flow for example when the test site is closer to the center of the contact interface 14, where the biological sensor 13 is disposed, than a predetermined range of the contact interface 14. The controller 15 judges that the test site is not at a suitable position when the test site is at a position farther from the center than the predetermined range. The controller 15 provides notification, via the notification interface 12, of information related to whether the contact position of the test site is a position suitable for measuring the amount of blood flow.

When the contact position of the test site is not a position suitable for measuring the amount of blood flow, the controller 15 calculates the direction and the distance the subject should move the test site to place the test site at a suitable position. The controller 15 then provides notification of the calculated result via the notification interface 12. Upon the subject confirming the notification and adjusting the position of the test site, the touch panel 113 detects movement of the test site and notifies the controller 15 of information related to the contact position of the test site. Based on the information related to the contact position acquired from the touch panel 113, the controller 15 judges whether the test site is at a position suitable for measuring the amount of blood flow.

When judging that the test site is not at a position suitable for measuring the amount of blood flow, the controller 15 calculates the direction and the distance the subject should move the test site to place the test site at a suitable position and provides notification of the calculated result via the notification interface 12. On the other hand, when judging that the test site is at a position suitable for measuring the amount of blood flow, the controller 15 provides notification, via the notification interface 12, indicating that the position of the test site is suitable.

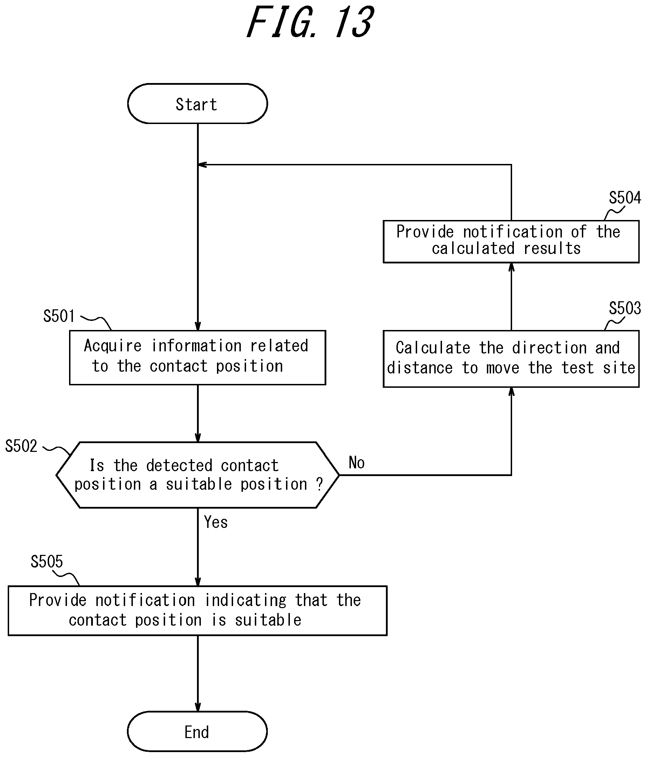

Next, with reference to the flowchart in FIG. 13, an example of the contact position adjustment processing that is executed by the controller 15 in Embodiment 4 is described. The controller 15 begins the processing flow in FIG. 13 for example when the measurement apparatus 10 is placed in a state capable of measuring the biological information by the subject operating the measurement apparatus 10.

The controller 15 acquires information related to the contact position detected by the touch panel 113 (step S501).

The controller 15 judges whether the contact position of the test site detected by the touch panel 113 is a position suitable for measuring the amount of blood flow (step S502).

When judging that the contact position of the test site is not a suitable position (step S502: No), the controller 15 calculates the direction and distance to move the test site to place the test site at a suitable position (step S503).

The controller 15 then provides notification, via the notification interface 12, of the result calculated in step S503 (step S504). After confirming the notification, the subject moves the test site based on the content of the notification, thereby making it easier to place the test site at a suitable position. Processing then transitions to step S501.

Upon judging, in step S502, that the contact position of the test site is a suitable position (step S502: Yes), the controller 15 provides notification, via the notification interface 12, indicating that the contact position of the test site is suitable (step S505) and terminates the processing.

Once the contact position of the test site is adjusted by the processing in FIG. 13, the controller 15 starts the processing for measurement illustrated in FIG. 5 and measures the biological information.

In this way, in the measurement apparatus 10 according to Embodiment 4, the controller 15 judges the contact state of the test site based on the contact position of the test site detected by the touch panel 113. When judging that the contact state is not suitable, the controller 15 can provide notification, via the notification interface 12, of the direction and distance to move the test site. Therefore, the subject can adjust the position of the test site in accordance with the notification, making it easier to adjust the contact state of the test site to a position suitable for measuring the biological information. In this way, the measurement apparatus 10 can improve the measurement accuracy of the biological information.

This disclosure is not limited to the above embodiments, and a variety of modifications and changes are possible. For example, the functions and the like included in the various components and steps may be reordered in any logically consistent way. Furthermore, components or steps may be combined into one or divided.

For example, in Embodiment 1, the controller 15 has been described as judging that the contact state is suitable when the illuminance value measured by the illuminance measurement unit 111 is less than the first illuminance threshold, but the judgment that the controller 15 makes about the contact state is not limited to this example. The controller 15 may, for example, judge the contact state based on a fourth illuminance threshold that is higher than the first illuminance threshold. For example, when the illuminance value measured by the illuminance measurement unit 111 is equal to or greater than the fourth illuminance threshold, the controller 15 judges that there is a large amount of external light incident from the contact interface 14 and that the contact interface 14 is not sufficiently covered by the test site. In this case, the controller 15 judges that the contact pressure of the test site on the contact interface 14 is weak and provides notification, via the notification interface 12, indicating to increase the contact pressure. The fourth illuminance threshold is, for example, stored in the memory 16.

In Embodiment 1, the controller 15 may judge the contact state of the test site based on an image captured by a camera that is the illuminance measurement unit 111. In greater detail, the controller 15 can judge the positional relationship between the test site and the biological sensor 13 based on the position of the test site captured by the camera. The controller 15 may judge the contact pressure of the test site based on the area of the test site captured by the camera.

In Embodiment 2, the controller 15 may further judge the contact state based on a fifth illuminance threshold that is lower than the second illuminance threshold. The controller 15 may judge whether the illuminance values measured by the four illuminance measurement units 111 are all less than the fifth illuminance threshold. When the controller 15 judges that the illuminance values measured by the four illuminance measurement units 111 are all less than the fifth illuminance threshold, the contact pressure is strong, and the capillaries at the test site are crushed. Therefore, the controller 15 judges that the contact state is not suitable for measuring the biological information. In this case, the controller 15 may provide notification, via the notification interface 12, indicating to lessen the contact pressure. The fifth illuminance threshold is, for example, stored in the memory 16.

In Embodiment 3, the controller 15 may further judge the contact state based on a third strain amount threshold that is larger than the first strain amount threshold. The controller 15 may judge whether the strain amounts detected by the four strain sensors 112 are all equal to or greater than the third strain amount threshold. When the controller 15 judges that the strain amounts detected by the four strain sensors 112 are all equal to or greater than the third strain amount threshold, the contact pressure is strong, and the capillaries at the test site are crushed. Therefore, the controller 15 judges that the contact state is not suitable for measuring the biological information. In this case, the controller 15 may provide notification, via the notification interface 12, indicating to lessen the contact pressure. The third illuminance threshold is, for example, stored in the memory 16.

In Embodiment 2, the contact interface 14 has been described as being disk-shaped, and the illuminance measurement units 111 have been described as being disposed around the periphery of the contact interface 14, but the shape of the contact interface 14 and the arrangement of the illuminance measurement units 111 are not limited to these examples. The contact interface 14 may have a different shape than a disk. Furthermore, the illuminance measurement units 111 may be disposed at any positions that allow judgment of suitability for measurement of the biological information in terms of the positional relationship with the biological sensor 13. For example, the contact interface 14 may be rectangular, and the illuminance measurement units 111 may be disposed to lie on a common circle, with the biological sensor 13 at the center of the circle. The same is true for the shape of the contact interface 14 and the arrangement of the strain sensors 112 in Embodiment 3. The touch panel 113 in Embodiment 4 as well may be any shape other than a disk.

In Embodiments 1 to 4, an example of the contact state detector 11 being the illuminance measurement units 111, strain sensors 112, or touch panel 113 has been described, but these examples are not exhaustive. The contact state detector 11 may have any configuration that can detect the contact state of the test site on the contact interface 14. The contact state detector 11 may, for example, be configured by a temperature sensor, such as a thermocouple. In this case, the controller 15 judges the contact state based on the temperature change detected by a plurality of temperature sensors.

In Embodiments 1 to 4, examples have been described in which the contact state detector 11 is of only one type, i.e. illuminance measurement units 111, strain sensors 112, or the touch panel 113, but the contact state detector 11 may be configured by a combination of illuminance measurement units 111, strain sensors 112, and the touch panel 113. The contact state detector 11 may, for example, be configured by two illuminance measurement units 111 and two strain sensors 112, or a portion may be configured by strain sensors 112 with the remainder being configured by the touch panel 113. In this way, the contact state detector 11 can be configured by combining an appropriate number and arrangement of the illuminance measurement units 111, the strain sensors 112, and the touch panel 113.

In Embodiments 1 to 4, the controller 15 may also judge the contact state of the test site continuously while the biological sensor 13 is acquiring the biological information. While the biological sensor 13 is acquiring the biological information, the controller 15 may suspend emission of laser light from the emitter 21 upon judging that the contact state is no longer appropriate. In this way, the controller 15 can suppress unnecessary power consumption by the measurement apparatus 10.

In Embodiments 1 to 4, the controller 15 provided in the measurement apparatus 10 has been described as generating the biological information based on output of the light receiver 22, but the biological information is not limited to being generated by the controller 15 provided in the measurement apparatus 10. For example, a server that is connected to the measurement apparatus 10 by a network that is wired, wireless, or a combination of both may be provided with a functional unit corresponding to the controller 15, and the server that includes this functional unit may generate the biological information. In this case, the measurement apparatus 10 acquires the biological measurement output via the biological sensor 13 and transmits the acquired biological measurement output to the server via a separately provided communication interface. The server generates the biological information based on the biological measurement output and transmits the generated biological information to the measurement apparatus 10. The user can view the biological information received by the measurement apparatus 10 by causing a display device to display the biological information. When the server generates biological information in this way, the measurement apparatus 10 can, for example, be reduced in size as compared to when all of the functional units in FIG. 1 are implemented on one measurement apparatus 10.

### REFERENCE SIGNS LIST

- 10: Measurement apparatus
- 11: Contact state detector
- 12: Notification interface
- 13: Biological sensor
- 14: Contact interface
- 15: Controller
- 16: Memory
- 17: Base
- 18: Support plate
- 20: Housing
- 21: Emitter
- 22: Light receiver
- 111: Illuminance measurement unit
- 112: Strain sensor
- 113: Touch panel

## Claims

1. A measurement apparatus for measuring biological information by contacting a test site to a contact interface, the measurement apparatus comprising:
a contact state detector configured to detect a contact state of the test site on the contact interface;
a biological sensor configured to acquire biological information from the test site;
a notification interface; and
a controller configured to measure the biological information based on output from the biological sensor;
wherein the controller judges the contact state of the test site on the contact interface based on output from the contact state detector and provides notification, via the notification interface, of information related to the contact state.

2. The measurement apparatus of claim 1, wherein the controller is further configured to activate the biological sensor upon judging that the contact state is suitable for measuring the biological information.

3. The measurement apparatus of claim 1, wherein the information related to the contact state includes information related to pressure with which the test site contacts the contact interface and/or information related to a positional relationship between the test site and the contact interface.

4. The measurement apparatus of claim 1, wherein the contact state detector comprises an illuminance measurement unit; and
the controller judges the contact state based on output of information related to an illuminance value measured by the illuminance measurement unit.

5. The measurement apparatus of claim 4, wherein a plurality of the illuminance measurement units is disposed around the biological sensor in the measurement apparatus; and
the controller judges the contact state based on output of information related to the illuminance value measured by each of the plurality of illuminance measurement units.

6. The measurement apparatus of claim 5, wherein the plurality of illuminance measurement units is disposed to lie on a common circle, with the biological sensor at the center of the circle.

7. The measurement apparatus of claim 5, wherein when the illuminance value measured by each of the plurality of illuminance measurement units is equal to or greater than a predetermined illuminance threshold, the controller provides notification, via the notification interface, indicating to increase pressure with which the test site contacts the contact interface.

8. The measurement apparatus of claim 5, wherein when the illuminance value measured by any of the plurality of illuminance measurement units is less than a predetermined illuminance threshold, the controller identifies an illuminance measurement unit, among the plurality of illuminance measurement units, measuring a highest illuminance value and provides notification, via the notification interface, indicating to move the test site towards the identified illuminance measurement unit.

9. The measurement apparatus of claim 1, wherein the contact state detector comprises a plurality of strain sensors around the biological sensor; and
the controller judges the contact state based on output of information related to a strain amount of the contact interface detected by each of the plurality of strain sensors.

10. The measurement apparatus of claim 9, wherein when the strain amount detected by each of the plurality of strain sensors is less than a predetermined strain amount threshold, the controller provides notification, via the notification interface, indicating to increase pressure with which the test site contacts the contact interface.

11. The measurement apparatus of claim 9, wherein when the strain amount detected by any of the plurality of strain sensors is equal to or greater than a predetermined strain amount threshold, the controller identifies a strain sensor, among the plurality of strain sensors, detecting a smallest strain amount and provides notification, via the notification interface, indicating to move the test site towards the identified strain sensor.

12. The measurement apparatus of claim 1, wherein the contact state detector comprises a touch panel; and
the controller judges the contact state based on output of information related to a contact position of the test site, the contact position being detected by the touch panel.

13. The measurement apparatus of claim 1, wherein the biological information includes information related to blood flow.

14. A measurement method for measuring biological information by contacting a test site to a contact interface, the method comprising:
detecting, with a contact state detector, a contact state of the test site on the contact interface;
judging, with a controller, the contact state of the test site on the contact interface based on output from the contact state detector and providing notification, with the controller via a notification interface, of information related to the contact state;
acquiring, with a biological sensor, biological information from the test site; and
measuring, with the controller, the biological information based on output from the biological sensor.
